(19) Europäisches Patentamt
European Patent Office
Office européen des brevets

(11) Veröffentlichungsnummer: **0 225 472**
**A1**

(12) # EUROPÄISCHE PATENTANMELDUNG

(21) Anmeldenummer: **86115107.4**

(22) Anmeldetag: **31.10.86**

(51) Int. Cl.⁴: **C07D 231/46**, C07D 405/06, C07D 409/04, C07D 405/04, C07C 131/00, A01N 43/56

(30) Priorität: **12.11.85 DE 3539995**

(43) Veröffentlichungstag der Anmeldung: **16.06.87 Patentblatt 87/25**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Jelich, Klaus, Dr.**
**Pahlkestrasse 5**
**D-5600 Wuppertal(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58a**
**D-5090 Leverkusen 3(DE)**
Erfinder: **Reinecke, Paul Dr.**
**Steinstrasse 8**
**D-5090 Leverkusen 3(DE)**

(54) **Substituierte Pyrazolin-5-one.**

(57) Substituierte Pyrazolin-5-one der Formel (I)

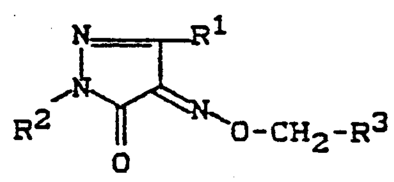

(I)

in welcher
$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegebenenfalls substituiertes Oxiranylalkyl, Aralkyl, Heterocyclyl oder Aryl stehen und
$R^3$ für Halogenalkyl, Halogenalkenyl, Halogenalkinyl, für substituiertes Cycloalkyl oder für substituiertes Cycloalkylalkyl steht,
haben und ihre Verwendung als Schädlingsbekämpfungsmittel, vor allem als Fungizide.

Sie können z.B. hergestellt werden, in dem man geeignete 4-Oximino-pyrazolin-5-one alkyliert oder geeignete Alkoximinocarbonsäureester mit geeigneten Hydrazin-Derivaten umsetzt.

EP 0 225 472 A1

## Substituierte Pyrazolin-5-one

Die Erfindung betrifft neue substituierte Pyrazolin-5-one, mehrere Verfahren zu ihrer Herstellung und ihre Verwendung als Schädlingsbekämpfungsmittel.

Es ist bereits bekannt, daß organische Stickstoff-Verbindungen wie beispielsweise das Zink-ethylen-1,2-bis-(dithiocarbamat) fungizide Eigenschaften besitzen (vgl. z.B. R. Wegler "Chemie der Pflanzenschutz-und Schädlingsbekämpfungsmittel", Springer Verlag Berlin, Heidelberg, New York 1970, Band 2, S. 65 f).

Die Wirkung dieser Verbindungen ist jedoch insbesondere bei niedrigen Aufwandmengen und -konzentrationen nicht immer in allen Anwendungsbereichen völlig zufriedenstellend.

Es wurden neue substituierte Pyrazolin-5-one der allgemeinen Formel (I)

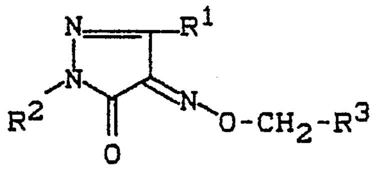

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegenbenenfalls substituiertes Oxiranylalkyl, Aralkyl, Heterocyclyl oder Aryl stehen und

$R^3$ für Halogenalkyl, Halogenalkenyl, Halogenalkinyl, für substituiertes Cycloalkyl oder für substituiertes Cycloalkylalkyl steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyrazolin-5-one der allgemeinen Formel -(I)

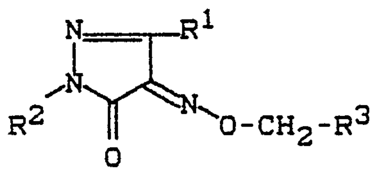

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegebenenfalls substituiertes Oxiranylalkyl, Aralkyl, Heterocyclyl oder Aryl stehen und

$R^3$ für Halogenalkyl, Halogenalkenyl, Halogenalkinyl, für substituiertes Cycloalkyl oder für substituiertes Cycloalkylalkyl steht,

erhält, wenn man

(a) 4-Oximino-pyrazolin-5-one der Formel (II)

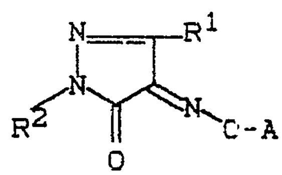

(II)

in welcher
$R^1$ und $R^2$ die oben angegebene Bedeutung haben und
A für Wasserstoff oder ein Alkalimetallkation steht,
mit Alkylierungsmitteln der Formel (III)
$R^3$-$CH_2$-X (III)
in welcher
$R^3$ die oben angegebene Bedeutung hat und
X für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt, oder wenn man
(b) Alkoximinocarbonsäureester der Formel (IV),

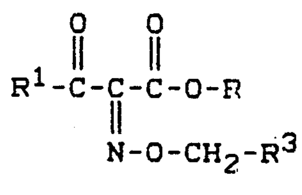

(IV)

in welcher
R für Alkyl steht und
$R^1$ und $R^3$ die oben angegebenen Bedeutungen haben,
mit Hydrazin-Derivaten der Formel (V)
$R^2$ -NH -$NH_2$ (V)
in welcher
$R^2$ die oben angegebene Bedeutung hat
gegebenenfalls in Gegenwart eines Verdünnungsmittels umsetzt,
oder wenn man
(c) die nach Verfahren (a) oder nach Verfahren (b) erhältlichen 4-Alkoxyimino-pyrazolin-5-one der Formel - (Ia),

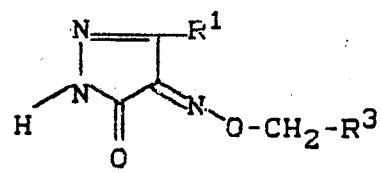

(Ia)

in welcher
$R^1$ und $R^3$ die oben angegebenen Bedeutungen haben,
mit Alkylierungsmitteln der Formel (VI)
$R^{2'}$-Y (VI)
in welcher
$R^{2'}$ für Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl oder für jeweils gegebenenfalls substituiertes Aralkyl oder Heterocyclyl steht und
Y für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen substituierten Pyrazolin-5-one der Formel (I) als Schädlingsbekämpfungsmittel, vor allem als Fungizide, verwendet werden können. Überraschenderweise zeigen die neuen substituierten Pyrazolin-5-one der Formel (I) bessere fungizide Eigenschaften als das aus dem Stand der Technik bekannte Zinkethylen-1,2-bis-dithiocarbamat, welches wirkungsmäßig eine naheliegende Verbindung ist.

Alkyl bedeutet in den Definitionen von $R^1$ und $R^2$ geradkettiges oder verzweigtes Alkyl mit 1 bis 8, vorzugsweise 1 bis 4 Kohlenstoffatomen. Es seien genannt Methyl, Ethyl, n-, i-Propyl, n-, s-, t-und i-Butyl.

Alkenyl bedeutet in den Definitionen von $R^1$ und $R^2$ Alkenyl mit bis zu 8, vorzugsweise bis zu 4 Kohlenstoffatomen.

Cyanalkyl bedeutet in den Definitionen von $R^1$ und $R^2$ Cyanalkyl mit 1 bis 8, insbesondere 1 oder 2 Kohlenstoffatomen im Alkylteil.

Hydroxyalkyl bedeutet in den Definitionen von $R^1$ und $R^2$ Hydroxyalkyl mit 1 bis 8, insbesondere 1 oder 2 Kohlenstoffatomen im Alkylteil.

Alkoxyalkyl bedeutet in den Definitionen von $R^1$ und $R^2$ Alkoxyalkyl mit 1 bis 8, insbesondere 1 oder 2 Kohlenstoffatomen je Alkoxy-und Alkylteil.

Alkylthioalkyl bedeutet in den Definitionen von $R^1$ und $R^2$ Alkylthioalkyl mit 1 bis 8, insbesondere 1 oder 2 Kohlenstoffatomen je Alkylthio-und Alkylteil.

Alkoxycarbonyl bedeutet in den Definitionen von $R^1$ und $R^2$ Alkoxycarbonyl mit 1 bis 8, insbesondere 1 oder 2 Kohlenstoffatomen im Alkoxyteil.

Hydroxycarbonylalkyl bedeutet in den Definitionen von $R^1$ und $R^2$ einen Rest mit 1 bis 8, insbesondere 1 oder 2 Kohlenstoffatomen im Alkylteil.

Alkoxycarbonylalkyl bedeutet in den Definitionen von $R^1$ und $R^2$ einen Rest mit 1 bis 8, insbesondere 1 oder 2 Kohlenstoffatomen je Alkoxy-und Alkylrest.

Aminocarbonylalkyl bedeutet in den Definitionen von $R^1$ und $R^2$ einen Rest mit 1 bis 8, insbesondere 1 oder 2 Kohlenstoffatomen je Alkylteil.

Mono-und Dialkylaminocarbonylalkyl bedeuten in den Definitionen von $R^1$ und $R^2$ einen Rest mit 1 bis 8, insbesondere 1 oder 2 Kohlenstoffatomen je Alkylrest.

Substituiertes Oxiranylalkyl bedeutet in den Definitionen von $R^1$ und $R^2$ gegebenenfalls durch Alkyl mit 1 bis 4, insbesondere 1 oder 2 Kohlenstoffatomen, substituiertes Oxiranylalkyl mit 1 bis 4, vorzugsweise 1 oder 2 Kohlenstoffatomen im Alkylteil.

Aralkyl bedeutet in den Definitionen von $R^1$ und $R^2$ einen Rest mit 6 bis 10 Kohlenstoffatomen, insbesondere 6, im Arylteil und 1 bis 4 Kohlenstoffatomen, insbesondere 1 oder 2 Kohlenstoffatomen im Alkylteil.

Heterocyclyl bedeutet in den Definitionen von $R^1$ und $R^2$ einen 5-oder 6-gliedrigen Ring mit 1 bis 3 gleichen oder verschiedenen Heteroatomen, genannt seien Saugerstoff, Schwefel und Stickstoff.

Aryl bedeutet in den Definitionen von $R^1$ und $R^2$ einen Rest mit 6 bis 10, insbesondere 6 Kohlenstoffatomen.

Halogen als Substituent in den Arylresten in den Definitionen von $R^1$ und $R^2$ bedeutet Fluor, Chlor, Brom und Jod, insbesondere Fluor, Chlor und Brom.

Alkyl als Substituent in den Arylresten in den Definitionen von $R^1$ und $R^2$ bedeutet einen geradkettigen oder verzweigten Rest mit 1 bis 4 Kohlenstoffatomen.

Alkoxy als Substituent in den Arylresten in den Definitionen von $R^1$ und $R^2$ bedeutet einen Rest mit 1 bis 4, insbesondere 1 oder 2 Kohlenstoffatomen.

Alkylthio als Substituent in den Arylresten in den Definitionen von $R^1$ und $R^2$ bedeutet einen Rest mit 1 bis 4, insbesondere 1 oder 2 Kohlenstoffatomen.

Dioxyalkylen als Substituent in den Arylresten in den Definitionen von $R^1$ und $R^2$ bedeutet einen Rest mit 1 bis 4, insbesondere 1 oder 2 Kohlenstoffatomen.

Alkylcarbonyloxy als Substituent in den Arylresten in den Definitionen von $R^1$ und $R^2$ bedeutet einen Rest mit 1 bis 4, insbesondere 1 oder 2 Kohlenstoffatomen im Alkylteil.

Halogenalkyl als Substituent in den Arylresten in den Definitionen von $R^1$ und $R^2$ bedeutet einen Rest mit 1 bis 4, insbesondere 1 Kohlenstoffatom und 1 bis 9 gleichen oder verschiedenen Halogenatomen, insbesondere Chlor und Fluor.

Halogenalkoxy als Substituent in den Arylresten in den Definitionen von $R^1$ und $R^2$ bedeutet einen Rest mit 1 bis 4, insbesondere 1 Kohlenstoffatom und 1 bis 9 gleichen oder verschiedenen Halogenatomen, insbesondere Fluor.

Halogenalkylthio als Substituent in den Arylresten in den Definitionen von $R^1$ und $R^2$ bedeutet einen Rest mit 1 bis 4, insbesondere 1 Kohlenstoffatom und 1 bis 9 gleichen oder verschiedenen Halogenatomen, insbesondere Fluor.

Halogenalkyl bedeutet in der Definition von $R^3$ einen Rest mit 1 bis 12, insbesondere 1 bis 8 Kohlenstoffatomen und 1 bis 8, insbesondere 1 bis 5 gleichen oder verschiedenen Halogenatomen.

Halogenalkenyl bedeutet in der Definition von $R^3$ einen Rest mit 2 bis 12, insbesondere 3 bis 8 Kohlenstoffatomen und 1 bis 8, insbesondere 1 bis 5 gleichen oder verschiedenen Halogenatomen.

Halogenalkinyl bedeutet in der Definition von $R^3$ einen Rest mit 2 bis 12, insbesondere 3 bis 8 Kohlenstoffatomen und 1 bis 8, insbesondere 1 bis 5 gleichen oder verschiedenen Halogenatomen.

Halogen in den Halogenalkyl, Halogenalkenyl und Halogenalkinyl Definitionen von $R^3$ bedeutet Fluor, Chlor, Brom und Jod, vorzugsweise Fluor, Chlor und Brom, insbesondere Fluor und Chlor.

Cycloalkyl in der Definition von $R^3$ bedeutet einen Rest mit 3 bis 7, bevorzugt 3 bis 6, insbesondere 3 Kohlenstoffatomen.

Cycloalkylalkyl in der Definition von $R^3$ bedeutet einen Rest mit 3 bis 7, bevorzugt 3 bis 6, insbesondere 3 Kohlenstoffatomen im Cycloalkylteil und 1 bis 7, vorzugsweise 1 bis 3, insbesondere 1 Kohlenstoffatom im Alkylteil.

Halogen als Substituent in den Cycloalkylteilen in den Definitionen von $R^3$ bedeutet Fluor, Chlor, Brom und Jod, insbesondere Fluor und Chlor.

Alkyl als Substituent in den Cycloalkylteilen in den Definitionen von $R^3$ bedeutet einen geradkettigen oder verzweigten Rest mit 1 bis 4, insbesondere 1 Kohlenstoffatom.

Halogenalkyl als Substituent in den Cycloalkylteilen in den Definitionen von $R^3$ bedeutet einen Rest mit 1 bis 4, insbesondere 1 Kohlenstoffatom im Alkylteil und 1 bis 9, insbesondere 3 Halogenatomen, wie Fluor, Chlor, Brom und Jod, insbesondere Fluor.

Die erfindungsgemäßen substituierten Pyrazolin-5-one sind durch die Formel (I) allgemein definiert. Bevorzugt sind Verbindungen der Formel (I), bei welchen

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl-, bzw. Alkenyl-, Alkinyl-oder Alkoxyteilen, für gegegebenenfalls durch Alkyl mit 1 bis 4 Kohlenstoffatomen substituiertes Oxiranylalkyl mit 1 bis 4 Kohlenstoffatomen im geradkettigen oder verzweigten Alkylteil, für einen 5-oder 6-gliedrigen Heterocyclus mit 1 bis 3 gleichen oder verschiedenen Heteroatomen, wie Sauerstoff, Schwefel und Stickstoff, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

$R^3$ für jeweils geradkettiges oder verzweigtes Halogenalkyl, Halogenalkenyl oder Halogenalkinyl mit jeweils bis zu 12 Kohlenstoffatomen und 1 bis 8 gleichen oder verschiedenen Halogenatomen, insbesondere Fluor, Chlor oder Brom steht oder für jeweils einfach oder mehrfach, gleich oder verschieden substituiertes Cycloalkyl oder Cycloalkylalkyl mit 3 bis 7 Kohlenstoffatomen im Cycloalkylteil und gegebenenfalls 1 bis 7 Kohlenstoffatomen im geradkettigen oder verzweigten Alkylteil steht, wobei als Substituenten jeweils infrage kommen: Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen.

Besonders bevorzugt sind Verbindungen der Formel (I), bei welchen

$R^1$ und $R^2$ unabhängig voneinander jeweils für Wasserstoff, Methyl, Ethyl, n-oder i-Propyl, n-, i-, s-oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein-bis dreifach, gleich oder verschieden substituiertes Phenyl oder Benzyl stehen, wobei als Substituenten in Frage kommen: Fluor, Chlor, Brom, Iod, Cyano, Nitro, Hydroxy, Methyl, Ethyl, n-oder i-Propyl, n-, i-, s-oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für jeweils geradkettiges oder verzweigtes Halogenalkyl, Halogenalkenyl oder Halogenalkinyl mit jeweils bis zu 8 Kohlenstoffatomen und 1 bis 5 gleichen oder verschiedenen Halogenatomen, insbesondere Fluor oder Chlor steht, oder für jeweils ein-bis fünffach gleich oder verschieden durch Fluor, Chlor, Methyl und/oder Trifluormethyl substituiertes Cyclopropyl, Cyclopentyl, Cyclohexyl, Cyclopropylmethyl, Cyclopentylmethyl, Cyclohexylmethyl, Cyclopropylethyl, 2-Cyclopentylethyl oder 3-Cyclohexyl-n-propyl steht.

Insbesondere seien Verbindungen der Formel (I) genannt, bei welchen

$R^1$ für Wasserstoff, Methyl, Ethyl, n-oder i-Propyl, n-, i-, s-oder t-Butyl steht,

$R^2$ für Wasserstoff, Methyl, Ethyl, n-oder i-Propyl, n-, i-, s-oder t-Butyl, Allyl, Butenyl, Propargyl, Cyanmethyl, Cyanethyl, Hydroxymethy, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein-bis dreifach, gleich oder verschieden substituiertes Phenyl oder Benzyl steht, wobei als Substituenten in Frage kommen: Fluor, Chlor, Brom, Cyano, Nitro, Hydroxy, Methyl, Ethyl, n-oder i-Propyl, n-, i-, s-oder t-Butyl, Methoxy, Ethoxy, Dioxymethylen, Dioxy ethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für Halogenalkyl oder Halogenalkenyl mit jeweils bis zu 8 Kohlenstoffatomen und 1 bis 5 gleichen oder verschiedenen Halogenatomen steht oder

$R^1$ und $R^2$ die oben gegebenen Bedeutungen haben und

$R^3$ für ein-bis fünffach, gleich oder verschieden jeweils durch Fluor, Chlor, Methyl und Trifluormethyl substituiertes Cyclopropyl oder Cyclopropylmethyl steht.

Im einzelnen seien außer den bei den Herstellungsbeispielen genannten Verbindungen die folgenden substituierten Pyrazolin-5-one der allgemeinen Formel (I) genannt:

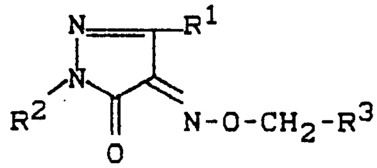

(I)

| $R^1$ | $R^2$ | $R^3$ | $R^1$ | $R^2$ | $R^3$ |
|---|---|---|---|---|---|
| -H | -H | $-CH=CCl_2$ | -H | $-CH_2CN$ | -C=CHCl<br>Cl |
| $-CH_3$ | -H | $-CH=CCl_2$ | $-CH_3$ | $-CH_3$ | -C=CHCl<br>Cl |
| $-CH_3$ | $-CH_3$ | $-CH=CCl_2$ | -H | $-CH_2CN$ | -CH=CH-Cl |
| $-CH_3$ | $-CH_2CN$ | $-CH=CCl_2$ | -H | $-CH_2$<br>$COOC_2H_5$ | -CH=CH-Cl |
| -H | $-CH_3$ | $-CH=CCl_2$ | -H | $-CH_2-CO$<br>$NH_2$ | -CH=CH-Cl |
| -H | $-CH_2CN$ | $-CH=CCl_2$ | -H | 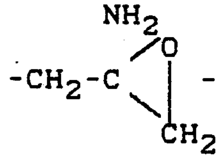 | -CH=CH-Cl |
| -H | -H | -C=CHCl<br>Cl | -H | $-CH_2CH_2OH$ | -CH=CH-Cl |
| -H | $-CH_3$ | -C=CHCl<br>Cl | $-CH_3$ | $-CH_2-CN$ | -CH=CHCl |

Verwendet man als Ausgangsstoffe beispielsweise 4-Hydroximino-1,3-dimethyl-pyrazolin-5-on und 2,2-Dichlorcyclopropylbrommethan, so läßt sich der Reaktionsablauf des erfindungsgemäßen Verfahrens (a) durch das folgende Formelschema darstellen:

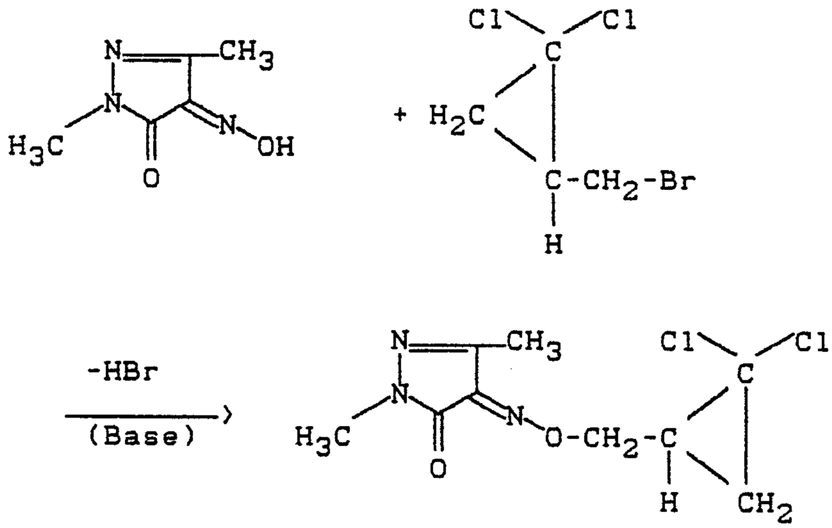


Verwendet man als Ausgangsstoffe beispielsweise β-Keto-α-[(2,2-dimethylcyclopropyl)-methoximino]-buttersäureethylester und Hydrazinhydrat, so läßt sich der Reaktionsablauf des erfindungsgemäßen Verfahrens (b) durch das folgende Formelschema darstellen:

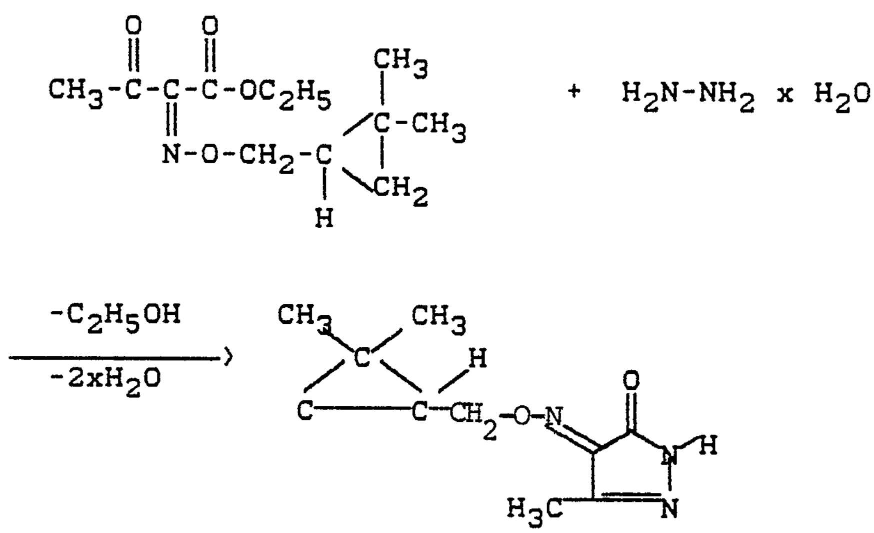


Verwendet man als Ausgangsstoffe beispielsweise 4-[(2,2-Dichlorcyclopropyl)-methoximino]-3-methyl-pyrazolin-5-on und Chloracetonitril, so läßt sich der Reaktionsablauf des erfindungsgemäßen Verfahrens (c) durch das folgende Formelschema darstellen:

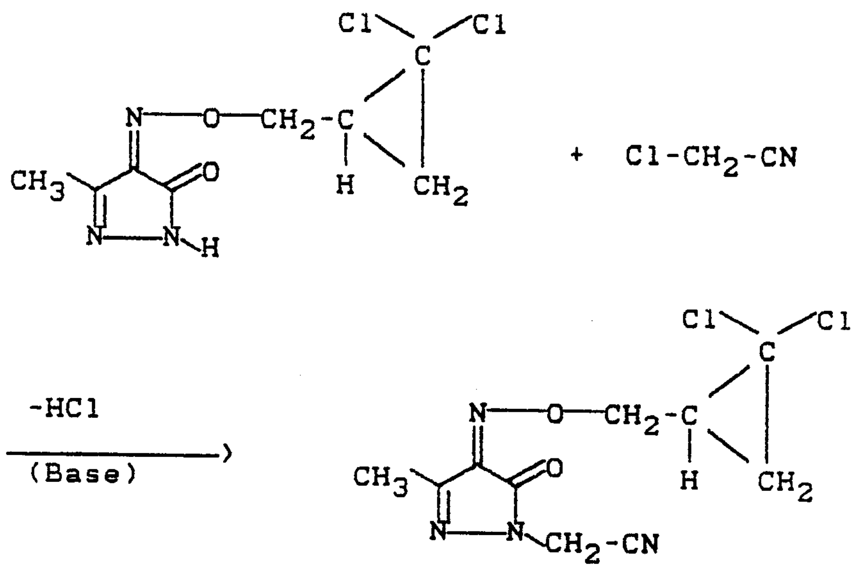


Die zur Durchführung des erfindungsgemäßen Verfahrens (a) als Ausgangsstoffe benötigten 4-Oximino-pyrazolin-5-one sind durch die Formel (II) allgemein definiert. in dieser Formel (II) stehen $R^1$ und $R^2$ für diejenigen Reste, die schon bei der Beschreibung der erfindungsgemäßen Stoffe der Formel (I) für diese Substituenten genannt wurden. A steht vorzugsweise für Wasserstoff oder für ein Natrium-oder Kaliumkation.

Die 4-Oximino-pyrazolin-5-one der Formel (II) sind teilweisebekannt [vgl. z.B. Ber. dtsch.chem.Ges. 29 , 249 (1896); Coll. Czech. Chem. Commun. 25, 55 (1960); Arch. Pharm. 309, 900 (1976); Liebigs Ann. Chem. 1976 , 1380].

Man erhält sie beispielsweise, wenn man $\beta$-Ketoester der Formel (VII)

$R^1-\underset{\underset{O}{\|}}{C}-CH_2-COOR^4$ (VII)

in welcher

$R^1$ die oben angegebene Bedeutung hat und

$R^4$ für niederes Alkyl, insbesondere für Methyl oder Ethyl steht,

oder wenn man Ethoxymethylenmalonester der Formel (VIII)

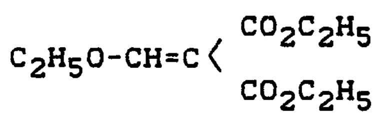

(VIII)

zunächst in einer 1. Stufe mit Hydrazinen der Formel (V)

$R^2-NH-NH_2$ (V)

in welcher

$R^2$ die oben angegebene Bedeutung hat,

gegebenenfalls in Gegenwart eines Verdünnungsmittels, wie beispielsweise Ethanol, bei Temperaturen zwischen 0° C und 100° C cyclisiert, die aus dem Malonester der Formel (VIII) sich ergebenden 4-Ethoxycarbonylpyrazolin-5-one der Formel (IX)

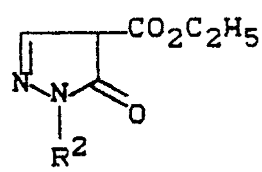

(IX).

in welcher

$R^2$ die oben angegebene Bedeutung hat,
in einem Zwischenschritt, nach üblichen Methoden, beispielsweise mit wäßriger Salzsäure bei Temperaturen zwischen 50° C und 120° C verseift und decarboxyliert, und die so erhältlichen Pyrazolin-5-one der Formel (X)

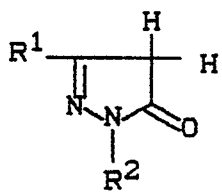

(X)

in welcher
$R^1$ und $R^2$ die oben angegebene Bedeutung haben,
in einer 2. Stufe (bzw. 3. Stufe) mit einem Nitrosierungsmittel, wie beispielsweise Isopentylnitrit oder Natriumnitrit, gegebenenfalls in Gegenwart eines Verdünnungsmittels, wie beispielsweise Ethanol, Wasser oder wäßriger Salzsäure und gegebenenfalls in Gegenwart einer Base, wie beispielsweise Natriummethylat, bei Temperaturen zwischen -20° C und +50° C umsetzt.

Die zur Durchführung des erfindungsgemäßen Verfahrens (a) als Ausgangsstoffe benötigten Alkylierungsmittel sind durch die Formel (III) allgemein definiert. In dieser Formel (III) steht $R^3$ für diejenigen Reste, die bereits im Zusammenhang mit der Beschreibung der erfindungsgemäßen Stoffe der Formel (I) für diese Substituenten genannt wurden. X steht vorzugsweise für Halogen, insbesondere Chlor, Brom oder Iod, oder gegebenenfalls substituiertes Alkylsulfonyloxy, Alkoxysulfonyloxy oder Arylsulfonyloxy, wie beispielsweise Methansulfonyloxy, Methoxysulfonyloxy, Trifluormethansulfonyloxy oder p-Toluolsulfonyloxy.

Die Alkylierungsmittel der Formel (III) sind allgemein bekannte Verbindungen der organischen Chemie.

Die β-Ketoester der Formel (VII) bzw. der Ethoxymethylenmalonester der Formel (VIII) sind ebenfalls allgemein bekannt.

Die zur Durchführung des erfindungsgemäßen Verfahrens (b) als Ausgangsstoffe benötigten Alkoximinocarbonsäureester sind durch die Formel (IV) allgemein definiert. In dieser Formel (IV) stehen $R^1$ und $R^3$ für diejenigen Reste, die bereits im Zusammenhang mit der Beschreibung der erfindungsgemäßen Stoffe der Formel (I) als bevorzugt für diese Substituenten genannt wurden. R steht vorzugsweise für geradkettiges oder verzweigtes Alkyl mit 1 bis 4 Kohlenstoffatomen, insbesondere für Methyl oder Ethyl.

Die Alkoximinocarbonsäureester der Formel (IV) sind teilweise bekannt (vgl. z.B. DE-OS 29 45 248; DE-OS 28 10 922; EP-OS 76 33 oder J. Antibiot. 36 , 846-854 [1983]).

Noch nicht bekannt sind Verbindungen der Formel (IVa),

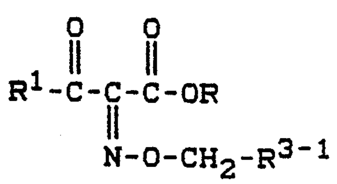

(IVa)

in welcher
R und $R^1$ die oben angegebene Bedeutung haben und
$R^{3-1}$ für Halogenalkenyl, Halogenalkinyl, für substituiertes Cycloalkyl oder für substituiertes Cycloalkylalkyl steht.

Man erhält sie in Analogie zu bekannten Verfahren, wenn man Hydroximinocarbonsäureester der Formel (XI),

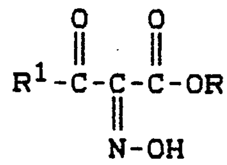

(XI)

in welcher

R und $R^1$ die oben angegebene Bedeutung haben,

mit Alkylierungsmitteln der Formel (IIIa),

$R^{3-1}$-$CH_2$-X (III a)

in welcher

$R^{3-1}$ die oben angegebene Bedeutung hat und

X für eine elektronenanziehende Abgangsgruppe, insbesondere für Chlor, Brom oder Iod, oder für gegebenenfalls substituiertes Alkylsulfonyloxy, Alkoxysulfonyloxy oder Arylsulfonyloxy wie beispielsweise Methansulfonyloxy, Methoxysulfonyloxy, Trifluormethansulfonyloxy oder p-Toluolsulfonyloxy steht,

gegebenenfalls in Gegenwart eines Verdünnungsmittels, wie beispielsweise Acetonitril, und gegebenenfalls in Gegenwart eines Säurebindemittels, wie beispielsweise Triethylamin, bei Temperaturen zwischen +10° C und +80° C umsetzt.

Die Alkylierungsmittel der Formel (IIIa) und die Hydroximinocarbonsäureester der Formel (XI) sind allgemein bekannte Verbindungen der organischen Chemie (vgl. z.B. Helv. Chim. Acta 67 , 906-915 [1984]; Franz. Patentanmeldung Nr. 2 434 572; Yakugaku Zasshi 87, 1209-1211 [1967] oder Chem. Ber. 100, 1245-1247 [1967]).

Die zur Durchführung des erfindungsgemäßen Verfahrens (c) als Ausgangsstoffe benötigten 4-Alkoximinopyrazolin-5-one sind durch die Formel (Ia) allgemein definiert. In dieser Formel (Ia) stehen $R^1$ und $R^3$ für diejenigen Reste, die bereits im Zusammenhang mit der Beschreibung der erfindungsgemäßen Stoffe der Formel (I) für diese Substituenten genannt wurden.

Die 4-Alkoximinopyrazolin-5-one der Formel (Ia) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^{2'}$ für diejenigen Reste, die bereits im Zusammenhang mit der Beschreibung der erfindungsgemäßen Stoffe der Formel (I) für den Substituenten $R^2$ genannt wurden mit Ausnahme des Wasserstoffrestes und der gegebenenfalls substituierten Arylreste. Y steht für diejenigen Abgangsgruppen, die bereits bei der Beschreibung der Alkylierungsmittel der Formel (III) für den Substituenten X genannt wurden.

Die Alkylierungsmittel der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel oder wäßrige Systeme in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid, oder auch Wasser oder wäßrig-organische Zweiphasen-Gemische, wie Dichlormethan-Wasser oder Toluol-Wasser.

Das erfindungsgemäße Verfahren (a) wird gegebenenfalls in Gegenwart eines Säurebindemittels durchgeführt. Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid; Alkalimetallcarbonate, wie Natriumcarbonat, Kaliumcarbonat oder Natriumhydrogencarbonat sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperatur kann bei der Durchführung des erfindungsgemäßen Verfahrens (a) in einem größeren Bereich variiert werden. Im allgemeinen arbeitet man bei Temperaturen zwischen -20° C und +200° C, vorzugsweise bei Temperaturen zwischen 0° C und +150° C.

Bei der Durchführung des erfindungsgemäßen Verfahrens (a) setzt man pro Mol 4-Oximino-pyrazolin-5-on der Formel (II) im allgemeinen 1,0 bis 3,0 Mol, vorzugsweise 1,0 bis 1,5 Mol, an Alkylierungsmittel der Formel (III) und gegebenenfalls 1,0 bis 3,0 Mol, vorzugsweise 1,0 bis 1,5 Mol, Säurebindemittel ein.

Führt man die Umsetzung in einem organisch-wäßrigen Zweiphasensystem durch, kann man gegebenenfalls in Gegenwart von 0,1 bis 1 Mol eines geeigneten Phasentransferkatalysators wie beispielsweise einer quartären Ammonium-oder Phosphoniumverbindung arbeiten. Beispielhaft seien Triethylbenzylammoniumchlorid und Benzyl-dodecyl-dimethylammoniumchlorid genannt.

Die Aufarbeitung und Isolierung der Reaktionsprodukte der Formel (I) erfolgt nach üblichen Methoden.

Als Verdünnungsmittel zur Durchführung des erfindungsgemäßen Verfahrens (b) kommmen ebenfalls inerte organische Lösungsmittel in Frage.
Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder-diethylether oder Alkohole, wie Methanol, Ethanol oder Propanol.

Die Reaktionstemperaturen können bei der Durchführung des erfindungsgemäßen Verfahrens (b) in einem größeren Bereich variiert werden. Im allgemeinen arbeitet man bei Temperaturen zwischen 0° C und 150° C, vorzugsweise bei Temperaturen zwischen 20° C und 120° C.

Zur Durchführung des erfindungsgemäßen Verfahrens (b) setzt man pro Mol Alkoximinocarbonsäureester der Formel (IV) im allgemeinen 0,8 bis 2,5 Mol, vorzugsweise 1,0 bis 1,2 Mol an Hydrazin-Derivat der Formel (V) ein. Die Reaktionsdurchführung, Aufarbeitung und Isolierung der Reaktionsprodukte der Formel (I) erfolgt nach üblichen Methoden.

Als Verdünnungsmittel zur Durchführung des erfindungsgemäßen Verfahrens (c) kommen ebenfalls inerte organische Lösungsmittel in Frage. Vorzugsweise verwendet man die bei Verfahren (a) angegebenen organischen Lösungsmittel oder wäßrig-organische Zweiphasen-Gemische.

Das erfindungsgemäße Verfahren (c) wird gegebenenfalls in Gegenwart eines Säurebindemittels durchgeführt. Vorzugsweise verwendet man als solche die bei Verfahren (a) angegebenen anorganischen oder organischen Basen.

Die Reaktionstemperaturen können bei der Durchführung des erfindungsgemäßen Verfahrens (c) ebenfalls in einem grösseren Bereich variiert werden. Im allgemeinen arbeitet man bei Temperaturen zwischen -20° C und +200° C, vorzugsweise bei Temperaturen zwischen 0° C und +150° C.

Bei der Durchführung des erfindungsgemäßen Verfahrens (c) setzt man pro Mol an 4-Alkoximino-pyrazolin-5-on der Formel (Ia) im allgemeinen 1,0 bis 3,0 Mol, vorzugsweise 1,0 bis 1,5 Mol an Alkylierungsmittel der Formel (VI) und gegebenenfalls 0,5 bis 3,0 Mol, vorzugsweise 0,6 bis 1,5 Mol an Säurebindemittel ein.

Die Reaktionsdurchführung, Aufarbeitung und Isolierung der Reaktionsprodukte der Formel (I) erfolgt, nach allgemein üblichen Verfahren.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel, vor allem als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;

Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei können die erfindungsgemäßen Wirkstoffe mit besonders gutem Erfolg zur Bekämpfung von Getreidekrankheiten wie beispielsweise gegen den Erreger der Braunspelzigkeit des Weizens (Leptosheria nodorum), gegen den Erreger der Streifenkrankheit der Gerste(Drechslera graminea), gegen Flugbrandkrankheit und gegen Fusarium-Arten, zur Bekämpfung von Gemüsekrankheiten wie beispielsweise gegen den Erreger der Tomatenbraunfäule (Phytophthora infestans) oder zur Bekämpfung von Reiskrankheiten wie beispielsweise gegen den Erreger der Reisfleckenkrankheit (Pyricularia oryzae) eingesetzt werden. Dabei zeigen die erfindungsgemäßen Wirkstoffe neben protektiver Wirksamkeit auch systemische Eigenschaften und lassen sich daher auch mit gutem Erfolg als Saatgutbeizmittel beispielsweise gegen den Erreger des echten Getreidemehltaus (Erysiphe graminis) verwenden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

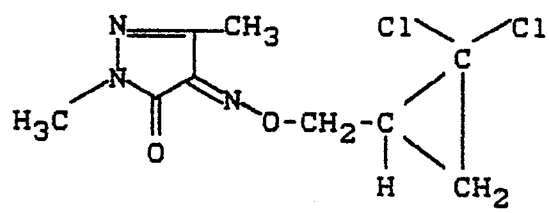

(Verfahren a)

Zu 4,64g (0,033 Mol) 1,3-Dimethyl-4-hydroximino-pyrazolin-5-on und 6,71g (0,033 Mol) 2,2-Dichlorcyclopropyl-brommethan in 80 ml Acetonitril gibt man bei Raumtemperatur 4,9 ml (0,035 Mol) Triethylamin und rührt 18 Stunden nach. Zur Aufarbeitung entfernt man das Lösungsmittel im Vakuum, nimmt den Rückstand in Dichlormethan auf, wäscht mit Wasser, trocknet über Natriumsulfat, entfernt das Lösungsmittel im Vakuum und reinigt den Rückstand chromatographisch an Kieselgel (Laufmittel = Dichlormethan/Ether; 10:1).

Man erhält 2,7g (31 % der Theorie) an 4-[(2,2-Dichlorcyclopropyl)-methoximino]-1,3-dimethyl-pyrazolin-5-on als Öl.

$^1$H-NMR ($CDCl_3$):$\delta$ = 4,3-4,8ppm.

Herstellung der Ausgangsverbindung

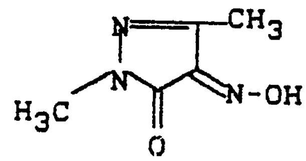

Zu 175 g (1,56 Mol) 1,3-Dimethyl-pyrazolin-5-on und 84,4 g (1,56 Mol) Natriummethylat in 1 l absolutem Ethanol gibt man unter Rühren und Eiskühlung tropfenweise 183 g (1,56 Mol) Isopentylnitrit, so daß die Innentemperatur 25° C bis 30° C nicht übersteigt. Nach beendeter Zugabe rührt man weitere 24 Stunden bei Raumtemperatur und saugt das ausgefallene Natriumsalz des 1,3-Dimethyl-4-hydroximino-pyrazolin-5-ons ab. Das kristalline Produkt wird in 1 l Wasser gelöst und mit Eisessig angesäuert. Zur vollständigen Fällung kühlt man für mehrere Stunden auf 0° C und saugt dann das Produkt ab.

Man erhält 162 g (74 % der Theorie) an 1,3-Dimethyl-4-hydroximino-pyrazolin-5-on vom Schmelzpunkt 93° C.

Beispiel 2:

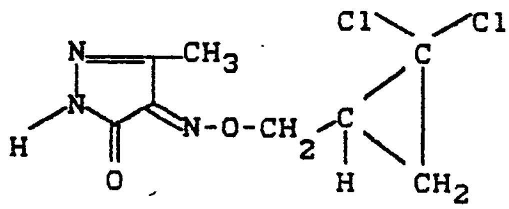

(Verfahren a)

Zu 11,43g (0,09 Mol) 3-Methyl-4-hydroximino-pyrazolin-5-on und 18,36g (0,09 Mol) 2,2-Dichlorcyclopropyl-brommethan in 100 ml Acetonitril gibt man bei Raumtemperatur 14 ml (0,1 Mol) Triethylamin und rührt 18 Stunden nach. Zur Aufarbeitung entfernt man das Lösungsmittel im Vakuum, nimmt den Rückstand in Dichlormethan auf, wäscht mit Wasser, trocknet über Natriumsulfat, entfernt das Lösungsmittel im Vakuum und reinigt den Rückstand chromatographisch an Kieselgel - (Laufmittel = Dichlormethan /Ether; 10:1). Man erhält 8,4g (37 % der Theorie) an 4-[(2,2-Dichlorcyclopropyl)-methoximino]-3-methyl-pyrazolin-5-on als Öl.

$^1$H-NMR ($CDCl_3$): $\delta$ = 4.33-4,8 ppm.

Beispiel 3

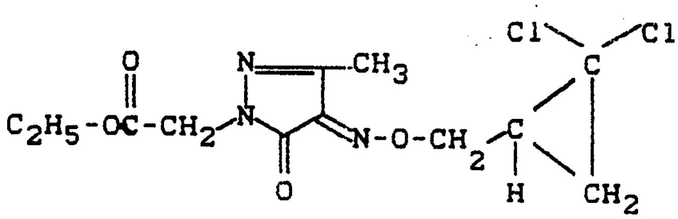

(Verfahren c)

3,3g (0,013 Mol) 4-[(2,2-Dichlor-cyclopropyl)-methoximino]-3-methyl-(1H)-pyrazolin-5-on und 2,5g (0,015 Mol) Bromessigsäureethylester werden zusammen mit 1,4g (0,01 Mol) Kaliumcarbonat in 50 ml Aceton 18 Stunden unter Rückfluß erhitzt. Zur Aufarbeitung entfernt man das Lösungsmittel im Vakuum, nimmt den Rückstand in Dichlormethan auf, wäscht mit Wasser, trocknet über Natriumsulfat, entfernt das Lösungsmittel im Vakuum und reinigt den Rückstand chromatographisch an Kieselgel - (Laufmittel = Dichlormethan/Ether; 10:1). Man erhält 3,7g (83 % der Theorie) an 4-[(2,2-Dichlorcyclopropyl)-methoximino]-1-ethoxycarbonylmethyl-3-methyl-pyrazolin-5-on als Öl.

$^1$H-NMR ($CDCl_3$): $\delta$ = 5,27ppm.

In entsprechender Weise und gemäß den allgemeinen Angaben zur Herstellung erhält man die substituierten Pyrazolin-5-one der allgemeinen Formel (I):

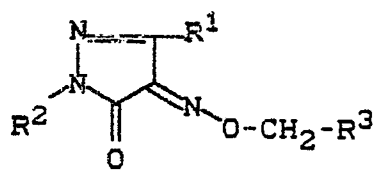

(I)

| Bsp. Nr. | $R^1$ | $R^2$ | $R^3$ | $^1H$-NMR*) |
|---|---|---|---|---|
| 4 | $CH_3$ | $CH_3$ | 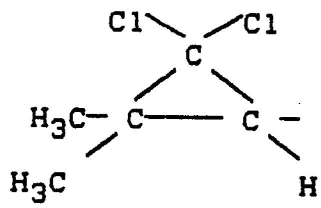 | 4,47-4,67 |
| 5 | H | $CH_3$ | 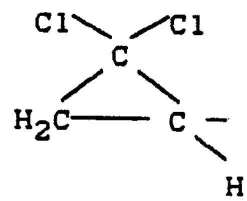 | 4,4-4,8 |
| 6 | H | $CH_3$ | 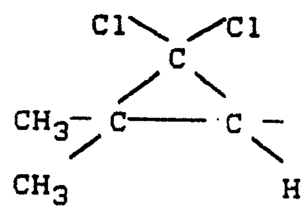 | 4,35-4,75 |
| 7 | $CH_3$ | H | 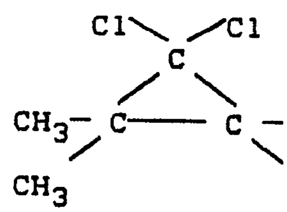 | 4,5-4,7 |
| 8 | $CH_3$ | $NC-CH_2-$ | 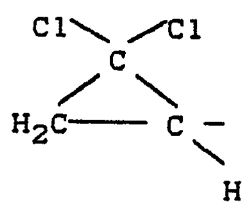 | 4,4-4,85 |
| 9 | $CH_3$ | $NC-CH_2-$ | 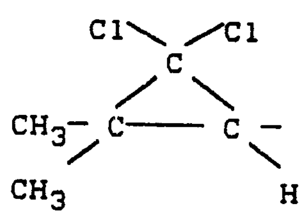 | 4,5-4,75 |

| Bsp. Nr. | $R^1$ | $R^2$ | $R^3$ | $^1H$-NMR*) |
|---|---|---|---|---|
| 10 | $CH_3$ | $C_2H_5OCO-CH_2-$ | 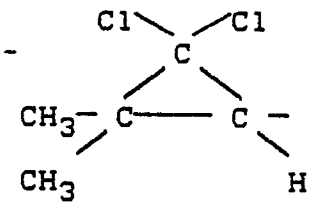 | 4,1-4,35 |
| 11 | H | $NC-CH_2CH_2-$ | 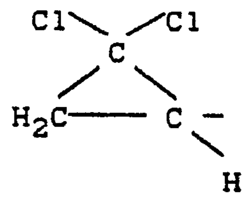 | 4,5-4,8 |
| 12 | H | $NC-CH_2CH_2-$ | 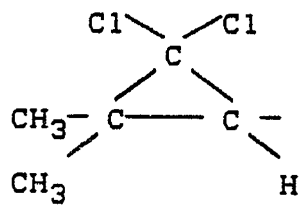 | 4,35-4,8 |
| 13 | $CH_3$ | $HO-CH_2CH_2-$ | 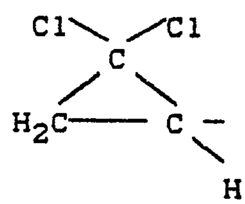 | 4,35-48 |
| 14 | $CH_3$ | $HO-CH_2CH_2-$ | 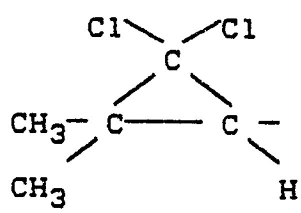 | 4,47-4,8 |
| 15 | $CH_3$ | $NC-CH_2CH_2-$ | 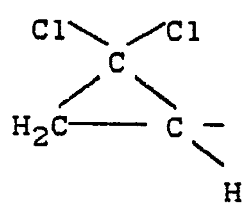 | 4,35-4,83 |

| Bsp. Nr. | $R^1$ | $R^2$ | $R^3$ | ¹H-NMR*) |
|---|---|---|---|---|
| 16 | $CH_3$ | $NC-CH_2CH_2-$ | 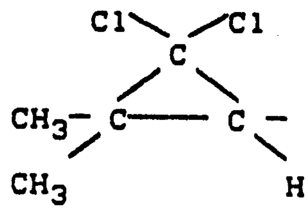 | 4,5-4,68 |
| 17 | $CH_3$ | $CH_3$ | Cl-CH=CH- | 4,87-4,99 |
| 18 | H | $CH_3$ | Cl-CH=CH- | 4,92-4,95 |
| 19 | H | $CH_3$ | Cl-CH=CH- | 4,92-4,98 (Isomeren-gemisch) |
| 20 | H | H | 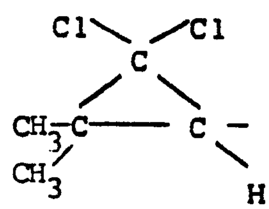 | 4,4-4,85 |
| 21 | H | H | 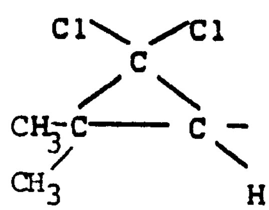 | 4,42-4,8 |
| 22 | H | H | Cl-CH=CH- | 4,87-4,93 |
| 23 | H | $NC-CH_2CH_2-$ | Cl-CH=CH- | 4,88-4,95 |
| 24 | $CH_3$ | $NC-CH_2CH_2-$ | Cl-CH=CH- | 4,92-5,0 |

| Bsp. Nr. | $R^1$ | $R^2$ | $R^3$ | ¹H-NMR*) |
|---|---|---|---|---|
| 25 | H | $CH_3$ | 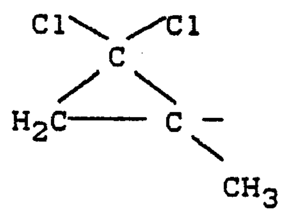 | 4,6-4,8 |
| 26 | $CH_3$ | $CH_3$ | 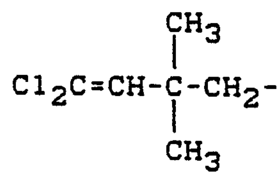 | 4,43-4,6 |
| 27 | H | $CH_3$ | 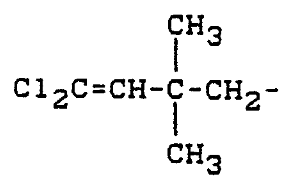 | 4,45-4,6 |
| 28 | H | $CH_3$ | 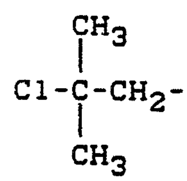 | 4,73-4,83 |
| 29 | $CH_3$ | $CH_3$ | 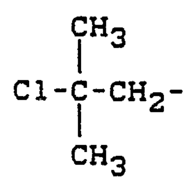 | 4,7-4,8 |
| 30 | $CH_3$ | $CH_3$ | 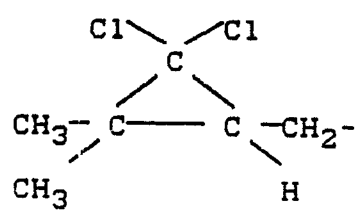 | 4,53-4,67 |

| Bsp. Nr. | $R^1$ | $R^2$ | $R^3$ | ¹H-NMR*) |
|---|---|---|---|---|
| 31 | H | $CH_3$ | 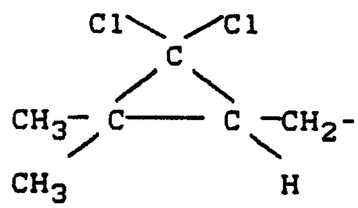 | 4,52-4,67 |
| 32 | $CH_3$ | $C_2H_5$ | 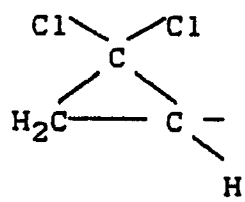 | 4,33-4,8 |
| 33 | $CH_3$ | $CH_3-(CH_2)_2-$ | 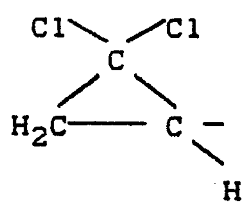 | 4,38-4,8 |
| 34 | $CH_3$ | $CH_3-(CH_2)_3-$ | 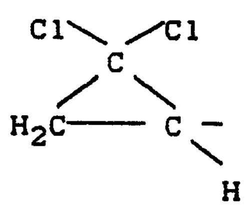 | 4,38-4,8 |
| 35 | $CH_3$ | $(CH_3)_2CH-$ | 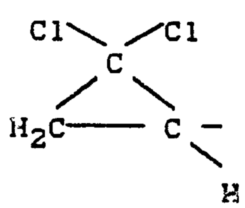 | 4,38-4,8 |

| Bsp. Nr. | $R^1$ | $R^2$ | $R^3$ | $^1$H-NMR*) |
|---|---|---|---|---|
| 36 | $CH_3$ | 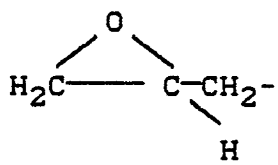 | 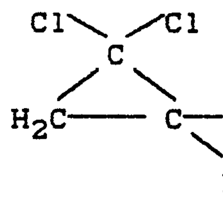 | 4,33-4,82 |
| 37 | H | $HO-CH_2-CH_2$ | 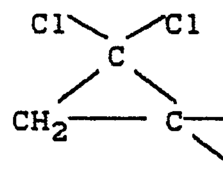 | 4,4-4,8 |

*) Die $^1$H-NMR-Spektren wurden aufgenommen in $CDCl_3$ mit Tetramethylsilan als innerem Standard. Angegebene sind in der Regel die chemischen Verschiebungen als δ-Werte für die Gruppierung $\rangle C=N-O-CH_2-$.

Anwendungsbeispiele

In den folgenden Anwendungsbeispielen wurde die nachstehend aufgeführte Verbindung als Vergleichssubstanz eingesetzt:

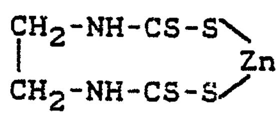

(A)

Zink-ethylen-1,2-bis-(dithiocarbamat)

Beispiel A

Phytophthora-Test (Tomate) / protektiv

Lösungsmittel:4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20° C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß Herstellungsbeispielen:
1, 5, 9, 16, 21 und 22.

Beispiel B

Leptosphaeria nodorum-Test (Weizen) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

Eine deutlich Überlegenheit in der Wirksamkeit gegenüber dem Stand der Tecknik zeigen bei diesem Test z.B. die Verbindungen gemäß den Herstellungsbeispielen:
1, 2, 3, 4, 6, 7, 8, 9, und 10.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

Eine deutliche Überlegenheit in der Wirksamkeit gegenüver dem Stand der Tecknik zeigen in diesem Test z.B. die Verbindungen gemäß den Herstellungsbeispielen:
7, 8, 9, 12, 14, 16 und 21.

**Ansprüche**

1. Substituierte Pyrazolin-5-one der Formel (I)

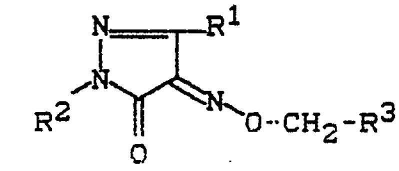

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegebenenfalls substituiertes Oxiranylalkyl, Aralkyl, Heterocyclyl oder Aryl stehen und

$R^3$ für Halogenalkyl, Halogenalkenyl, Halogenalkinyl, für substituiertes Cycloalkyl oder für substituiertes Cycloalkylalkyl steht.

2. Substituierte Pyrazolin-5-one gemäß Anspruch 1, wobei in der Formel (I)

$R^1$ und $R^2$ unabhängig voneinander jeweils für Wasser stoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl-, bzw. Alkenyl-, Alkinyl-oder Alkoxyteilen, für gegegebenenfalls durch Alkyl mit 1 bis 4 Kohlenstoffatomen substituiertes Oxiranylalkyl mit 1 bis 4 Kohlenstoffatomen im geradkettigen oder verzweigten Alkylteil, für einen 5-oder 6-gliedrigen Heterocyclus mit 1 bis 3 gleichen oder verschiedenen Heteroatomen, für gegebenenfalls einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen genannt seien: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

$R^3$ für jeweils geradkettiges oder verzweigtes Halogenalkyl, Halogenalkenyl oder Halogenalkinyl mit jeweils bis zu 12 Kohlenstoffatomen und 1 bis 8 gleichen oder verschiedenen Halogenatomen steht oder für jeweils einfach oder mehrfach, gleich oder verschieden substituiertes Cycloalkyl oder Cycloalkylalkyl mit 3 bis 7 Kohlenstoffatomen im Cycloalkylteil und gegebenenfalls 1 bis 7 Kohlenstoffatomen im geradkettigen oder verzweigten Alkylteil steht, wobei als Substituenten genannt seien: Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen.

3. Substituierte Pyrazolin-5-one gemäß Anspruch 1, wobei in der Formel (I)

$R^1$ und $R^2$ unabhängig voneinander jeweils für Wasserstoff, Methyl, Ethyl, n-oder i-Propyl, n-, i-, s-oder t-Butyl, Allyl, Butenyl, Propargyl, Cyanmethyl, Cyanethyl, Hydroxymethyl, Hydroxyethyl, Methoxymethyl, Methoxyethyl Ethoxymethyl, Ethoxyethyl, Methylthiomethyl, Methoxycarbonyl, Ethoxycarbonyl, Hydroxycarbonyl, Hydroxycar bonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein-bis dreifach, gleich oder verschieden substituiertes Phenyl oder Benzyl stehen, wobei als Substituenten genannt seien: Fluor, Chlor, Brom, Iod, Cyano, Nitro, Hydroxy, Methyl, Ethyl, n-oder i-Propyl, n-, i-, s-oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für jeweils geradkettiges oder verzweigtes Halogenalkyl, Halogenalkenyl oder Halogenalkinyl mit jeweils bis zu 8 Kohlenstoffatomen und 1 bis 5 gleichen oder verschiedenen Halogenatomen steht, oder für jeweils ein-bis fünffach, gleich oder verschieden durch Fluor, Chlor, Methyl und/oder Trifluormethyl substituiertes Cyclopropyl, Cyclopentyl, Cyclohexyl, Cyclopropylmethyl, Cyclopentylmethyl, Cyclohexylmethyl, Cyclopropylethyl, 2-Cyclopentylethyl oder 3-Cyclohexyl-n-propyl steht.

4. Substituierte Pyrazolin-5-one gemäß Anspruch 1, wobei in der Formel (I)

$R^1$ für Wasserstoff Methyl, Ethyl, n-oder i-Propyl, n-, i-, s-oder t-Butyl steht,

$R^2$ für Wasserstoff, Methyl, Ethyl, n-oder i-Propyl, n-, i-, s-oder t-Butyl, Allyl, Butenyl, Propargyl, Cyanmethyl, Cyanethyl, Hydroxymethy, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothen-3-yl oder für gegebenenfalls ein-bis dreifach, gleich oder verschieden substituiertes Phenyl oder Benzyl steht, wobei als Substituenten genannt seien: Fluor, Chlor, Brom, Iod, Cyano, Nitro, Hydroxy, Methyl, Ethyl, n-oder i-Propyl, n-, i-, s-oder t-butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für Halogenalkyl oder Halogenalkenyl mit jeweils bis zu 8 Kohlenstoffatomen und 1 bis 5 gleichen oder verschiedenen Halogenatomen steht.

5. Substituierte Pyrazolin-5-one gemäß Anspruch 1, wobei in der Formel (I)

$R^1$ für Wasserstoff, Methyl, Ethyl, n-oder i-Propyl, n-, i-, s-oder t-Butyl steht,

$R^2$ für Wasserstoff, Methyl, Ethyl, n-oder i-Propyl, n-, i-, s-oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein-bis dreifach, gleich oder verschieden substituiertes Phenyl oder Benzyl steht, wobei als Substituenten genannt seien: Fluor, Chlor, Brom, Iod, Cyano, Nitro, Hydroxy, Methyl, Ethyl, n-oder i-Propyl, n-, i-, s-oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

$R^3$ für ein-bis fünffach, gleich oder verschieden jeweils durch Fluor, Chlor, Methyl und Trifluormethyl substituiertes Cylopropyl oder Cyclopropylmethyl steht.

6. Verfahren zur Herstellung von substituierten Pyrazolin-5-onen der Formel (I)

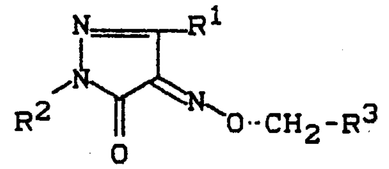

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegebenenfalls substituiertes Oxiranylalkyl, Aralkyl, Heterocyclyl oder Aryl stehen und

$R^3$ für Halogenalkyl, Halogenalkenyl, Halogenalkinyl, für substituiertes Cycloalkyl oder für substituiertes Cycloalkylalkyl steht,

dadurch gekennzeichnet, daß man

(a) 4-Oximino-Pyrazolin-5-one der Formel (II)

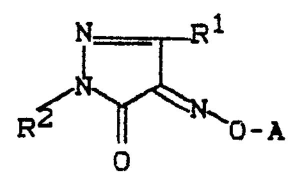

(II)

in welcher

$R^1$ und $R^2$ die oben angegebenen Bedeutungen haben und

A für Wasserstoff oder ein Alkalimetallkation steht,

mit Alkylierungsmitteln der Formel (III)

$R^3$-$CH_2$-X (III)

in welcher

$R^3$ die oben angegebene Bedeutung hat und
X für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt, oder daß man
(b) Alkoximinocarbonsäureester der Formel (IV),

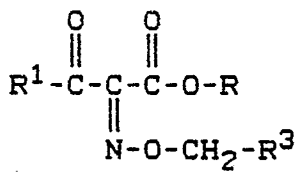

(IV)

in welcher
R für Alkyl steht und
$R^1$ und $R^3$ die oben angegebenen Bedeutungen haben, mit Hydrazin-Derivaten der Formel (V)
$R^2$ -NH -$NH_2$ (V)
in welcher $R^2$ die oben angegebene Bedeutung hat,
gegebenenfalls in Gegenwart eines Verdünnungsmittels umsetzt,
oder daß man
(c) die nach Verfahren (a) oder nach Verfahren (b) erhältlichen 4-Alkoxyimino-pyrazolin-5-one der Formel - (Ia),

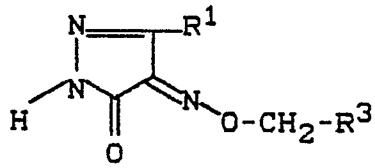

(Ia)

in welcher
$R^1$ und $R^3$ die oben angegebenen Bedeutungen haben, mit Alkylierungsmitteln der Formel (VI)
$R^{2'}$-Y (VI)
in welcher
$R^{2'}$ für Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl oder für jeweils gegebenenfalls substituiertes Aralkyl oder Heterocyclyl steht und
Y für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt.

7. Schädlingsbekämpfungsmittel, gekennzeichnet durch einen Gehalt an mindestens einem substituierten Pyrazolin-5-on der Formel (I) gemäß den Ansprüchen 1 bis 6.

8. Verwendung von substituierten Pyrazolin-5-onen der Formel (I) gemäß den Ansprüchen 1 bis 6 zur Bekämpfung von Schädlingen.

9. Verfahren zur Bekämpfung von Schädlichen, dadurch gekennzeichnet, daß man substituierte Pyrazolin-5-one der Formel (I) gemäß den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man substituierte Pyrazolin-5-one der Formel (I) gemäß den Ansprüchen 1 bis 6 mit Streckmitteln und/oder oberflächenaktiven Mitteln vermischt.

11. Oximinocarbonsäureester-Derivate der Formel (IVa)

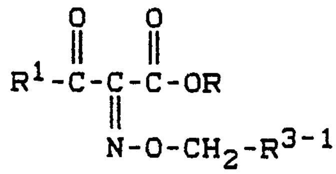 (IVa)

in welcher

R für Alkyl,

R' Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils gegebenenfalls substituiertes Oxiranylalkyl, Aralkyl, Heterocyclyl oder Aryl steht und

$R^{3-1}$ für Halogenalkenyl, Halogenalkinyl, für substituiertes Cycloalkyl oder substituiertes Cycloalkylalkyl steht.

Europäisches Patentamt

# EUROPÄISCHER RECHERCHENBERICHT

Nummer der Anmeldung

EP 86 11 5107

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-2 510 696 (B.A. HUNTER et al.)<br>* ganzes Dokument *<br>--- | 1,7-10 | C 07 D 231/46<br>C 07 D 405/06<br>C 07 D 409/04<br>C 07 D 405/04<br>C 07 C 131/00<br>A 01 N 43/56 |
| A | BEILSTEINS HANDBUCH DER ORGANISCHEN CHEMIE, 4. Auflage, Band 24, Verlag von Julius Springer, 1936, Berlin; "Verbindungen mit 2 cyclisch gebunden Stickstoffatomen mono- und poly-oxo-Verbindungen"<br>* Seite 391, Zeilen 1-4 *<br>--- | 1,6 | |
| A | CHEMICAL ABSTRACTS, Band 97, Nr. 5, 2. August 1982, Seite 569, Spalte 2, Zusammenfassungs Nr. 38878h, Columbus, Oho, US; T. UEDA et al.: "Synthesis of pyrazolone derivatives. XXXX. Synthesis and analgesic activity of 4-alkoxyimino (or alkylaminomethylene)-3-methylcarbamoyl-1-methyl(or phenyl)-2-pyrazolin-5-ones"; & Jakukaku ZASSHI, Band 102, Nr. 3, 1982, Seiten 295-299<br>--- | 1 | RECHERCHIERTE SACHGEBIETE (Int. Cl.4)<br>C 07 D 231/00<br>C 07 D 405/00<br>C 07 D 409/00<br>C 07 C 131/00<br>A 01 N 43/00 |
| A,D | FR-A-2 434 572 (CIBA-GEIGY A.G.)<br>* Anspruch 1 *<br>--- -/- | 11 | |

Der vorliegende Recherchenbericht wurde für alle Patentansprüche erstellt.

| Recherchenort | Abschlußdatum der Recherche | Prüfer |
|---|---|---|
| BERLIN | 13-02-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 86 11 5107

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP-A-0 166 171 (BAYER A.G.) * Ansprüche * ----- | 1,6-10 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prufer |
|---|---|---|
| BERLIN | 13-02-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82